# EUROPEAN PATENT APPLICATION

(11) **EP 0 960 940 A1**
(43) Date of publication of application: **01.12.1999**
(21) Application number: 98201636.2
(22) Date of filing: 19.05.1998
(51) Int. Cl.: C12N 15/82, C12N 15/33, C12N 5/10, A01H 5/00

(54) **Geminivirus inducible promoter sequences and the use thereof in geminivirus infected plants or plant cells**

(71) Applicant: Centro de Investigacion y de Estudios Avanzados del I.P.N.( CINVESTAV), 36500 Irapuato (MX)
(72) Inventor: The designation of the inventor has not yet been filed
(74) Representative: VOSSIUS & PARTNER

(57) **Abstract**

The present invention is generally directed to geminivirus inducible promoter sequences which are in itself normally, if present in a plant cell, not active for the expression of adjacent genes. However it appears that upon infection of the plant with wild-type virus, or a part thereof such as the AC2 protein, expression of said adjacent genes occurs. It appears that so-called CLE's, present in the geminiviral promoter, are necessary and sufficient to induce said expression. Thus it is feasible to construct transgenic plants which are resistant to geminiviral infection.

SEQUENCE LISTING

(1) GENERAL INFORMATION:

(i) APPLICANT:
(A) NAME: Centro de Investigación y de estudios
avanzados del I.P.N. (CINVESTAV)
(B) STREET: Apdo. Postal 629
(C) CITY: Irapuato
(D) STATE: none
(E) COUNTRY: Mexico
(F) POSTAL CODE (ZIP): 36500

(ii) TITLE OF INVENTION: Geminivirus inducible promoter sequences and
the use thereof in geminivirus infected plants or plant
cells

(iii) NUMBER OF SEQUENCES: 7

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98 20 1636.2

(2) INFORMATION FOR SEQ ID NO: 1:

(i) SEQUENCE CHARACTERISTICS:
(A) LENGTH: 119 base pairs
(B) TYPE: nucleic acid
(C) STRANDEDNESS: double
(D) TOPOLOGY: linear

(ii) MOLECULE TYPE: DNA

(iii) HYPOTHETICAL: NO

(xi) SEQUENCE DESCRIPTION: SEQ ID NO: 1:

GAAGCTTAGT TATCAGTTCC AGACGTGGGG ACCAAGTAGT GTATGACCAC TTTATTGACT 60

GTCAGCTTTA TAAATTGAAA TTAAAACATA AGTGGTCCAT GTACCTTTAA TTCAAAATG 119

(2) INFORMATION FOR SEQ ID NO: 2:

(i) SEQUENCE CHARACTERISTICS:
(A) LENGTH: 7 base pairs
(B) TYPE: nucleic acid
(C) STRANDEDNESS: double
(D) TOPOLOGY: linear

(ii) MOLECULE TYPE: DNA

(iii) HYPOTHETICAL: YES

(xi) SEQUENCE DESCRIPTION: SEQ ID NO: 2:

GTGGTCC 7

(2) INFORMATION FOR SEQ ID NO: 3:

(i) SEQUENCE CHARACTERISTICS:
(A) LENGTH: 6 base pairs
(B) TYPE: nucleic acid
(C) STRANDEDNESS: double
(D) TOPOLOGY: linear

(ii) MOLECULE TYPE: DNA

(iii) HYPOTHETICAL: YES

(xi) SEQUENCE DESCRIPTION: SEQ ID NO: 3:

GACCAC 6

(2) INFORMATION FOR SEQ ID NO: 4:

(i) SEQUENCE CHARACTERISTICS:
(A) LENGTH: 8 base pairs
(B) TYPE: nucleic acid
(C) STRANDEDNESS: double
(D) TOPOLOGY: linear

(ii) MOLECULE TYPE: DNA

(iii) HYPOTHETICAL: YES

(xi) SEQUENCE DESCRIPTION: SEQ ID NO: 4:

GGGACCAA 8

(2) INFORMATION FOR SEQ ID NO: 5:

(i) SEQUENCE CHARACTERISTICS:
(A) LENGTH: 42 base pairs
(B) TYPE: nucleic acid
(C) STRANDEDNESS: double
(D) TOPOLOGY: linear

(ii) MOLECULE TYPE: DNA

(iii) HYPOTHETICAL: YES

(xi) SEQUENCE DESCRIPTION: SEQ ID NO: 5:

AGCTTTAAAG TGGTCCCAAA GGCCTTGTGG TCCCAAATGA TC 42

(2) INFORMATION FOR SEQ ID NO: 6:

(i) SEQUENCE CHARACTERISTICS:
(A) LENGTH: 23 base pairs
(B) TYPE: nucleic acid
(C) STRANDEDNESS: double
(D) TOPOLOGY: linear

(ii) MOLECULE TYPE: DNA

(iii) HYPOTHETICAL: YES

(xi) SEQUENCE DESCRIPTION: SEQ ID NO: 6:

AGGCCTTGTG GTCCCAAATG ATC 23

(2) INFORMATION FOR SEQ ID NO: 7:

(i) SEQUENCE CHARACTERISTICS:
(A) LENGTH: 30 base pairs
(B) TYPE: nucleic acid
(C) STRANDEDNESS: double
(D) TOPOLOGY: linear

(ii) MOLECULE TYPE: DNA

(iii) HYPOTHETICAL: YES

(xi) SEQUENCE DESCRIPTION: SEQ ID NO: 7:

CGGCTGTTGA ATTTAAATAC ATATTAACTG 30

## Description

The present invention is generally directed to geminivirus inducible promoter sequences which are - in itself - normally, if present in a plant cell, not active for the expression of adjacent genes. However it appears that upon infection of the plant with wild-type virus, or a part thereof such as the AC2 protein, expression of said adjacent genes occurs under the control and influence of the geminiviral promoter. It appears that so-called CLE's, present in the geminiviral promoter, are necessary and sufficient to induce said expression.
According to the current invention it is thus feasible to construct transgenic plants, comprising at least one of said CLE's or functional fragments thereof, which are resistant to geminiviral infection. To obtain this effect, adjacent to or operably linked to any of the said CLE's any gene or gene combination can be constructed, which gene or gene product is able to interfere with the outbreak or growth characteristics of the geminivirus in order to arrest further spread of the geminivirus in the infected plant or part thereof.

Viruses are the causative agents of a large number of serious and potentially serious diseases in humans, animals and plant. Plant viruses in particular have the potential to destroy or reduce crop yield and to otherwise have a deleterious effect on agricultural and horticultural industries to economically significant levels.

Particularly important viruses in this regard are the DNA viruses, including the geminiviruses. The geminiviruses are a group of small DNA viruses which infect plant cells, They contain a single strand of circular DNA referred to as "virion-sense" or "positive-sense" DNA of less than about 2900 base pairs. Upon infection of a host cell by a geminivirus, the viral coat protein is removed and a double stranded replicative form of DNA is synthesised comprising the virion-sense strand and a "complementary-sense" strand. Transcription occurs from both the virion-sense strand and from the "complementary sense" strand, giving rise to (+) and (-) sense RNA transcripts respectively.

Examples of geminiviruses include Maize Streak Virus (MSV), Bean Golden Mosaic Virus (BGMV), African Cassava Mosaic Virus (ACMV), Chloris Striate Mosaic Virus (CSMV), Tomato Yellow Leaf Curl Virus (TYLCV), Tomato Golden Mosaic Virus (TGMV), Beet Curly Top Virus (BCTV) and Tomato Leaf Curl Virus (TLCV) amongst many others. These viruses cause economically important diseases in cultivated plants including leaf curling and other leaf distortion.

Geminiviruses replicate in the nucleus of the infected cell and are thought to employ a rolling-circle mechanism similar to one used by the single-stranded DNA containing coliphages and certain *Staphylococcus aureus* and *Bacillus subtilis* plasmids. The other known plant viruses, including other plant DNA viruses, replicate via RNA intermediates.

Geminivirus particles accumulate in the nuclei of infected cells where DNA replication and virus assembly probably take place (Davies *et al*, J. Cell. Sci (Suppl) (1987) 7 : 95-107). Their putative replicative forms are double-stranded covalently closed circular DNA of about 2,7 Kb in chromatine-like structures and are likely to be the transcriptionally active forms of the virus (Abouzid *et al,* Mol. Gen. Genet. (1988) 212 : 252-258).

Geminiviruses have been classified into three subgroups according to their host range (mono or dicotyledonous plants), insect vector (whiteflies or leafhoppers), and genome organization (mono- or bipartite). All bipartite geminiviruses described so far show similar genomic organization. Component A codes for all the viral proteins required for replication and transcription of the viral DNA : AC1 ( Rep), AC2 (TrAP) and AC3, and the coat protein AV1 (CP). The B component codes for the BC1 and BV1 proteins involved in the cell-to-cell and systemic movement of the virus in the plant. Both components display an intergenic region (IR) which includes a 180-200 nt segment almost identical in both components and known as the common region. All geminiviruses have an element with the potential to form a stem-loop structure. The entire structure (∼30 nt) is conserved among bipartite geminiviruses whereas in monopartite geminiviruses the sequence of the stem varies, conserving only the loop sequence (Lazarowitz, 1992).

Since geminiviruses are the only plant virus group as described above that replicate solely in the nucleus and without RNA intermediates, these features make them amenable for their use as plant expression vectors and as models for the study of plant gene regulation and DNA replication (Davies and Stanley, 1989; Lazarowitz, 1992; Timmermans *et al.,* 1994). The study of gene expression in animal viruses, such as SV40, adenovirus, herpesvirus, among many others, has offered important insights into the corresponding processes in their hosts. Accordingly, the geminiviruses might shed some light into the analogous mechanisms in plant cells.

In general, the expression of viral genes follows a temporal sequence that is finely coordinated. At the onset of infection the virus relies entirely on the host transcriptional machinery. However, some of the viral proteins synthesized in the first stage participate in the regulation of viral genes in subsequent stages (reviewed by Martin and Green, 1992; Nevins, 1991). In the case of geminiviruses, it has been reported that Rep downregulates its own expression (Haley *et al*., 1992: Sunter *et al,* 1993), whereas AC2 regulates the expression of virion-sense genes from both components, CP and BV1 (Sunter and Bisaro, 1991; Sunter and Bisaro, 1992). The amino acid sequence of AC2 displays features of an eukaryotic transactivator, including a basic domain associated with an atypical zinc-finger motif and a carboxy-terminal acidic domain. However, sequence-specific DNA binding of AC2 has not been demonstrated (Noris *et al,* 1996; Sung and Coutts, 1996). It has also been shown that heterologous complementation for the AC2 gene can occur among distantly-related geminiviruses, suggesting a conservation of the target sequence (Sunter *et al,* 1994). The mechanism by which AC2 activates viral genes and the *cis*-elements involved have not been elucidated. This might yield interesting information on the regulatory properties of this protein (one of the smallest transcriptional activators described) and also on sequences that could also be present within the plant genome.

The intergenic region (IR) of geminiviruses harbors two divergent, overlapping promoters (Lazarowitz, 1992). Even though the upstream limits of these promoters have not been defined, it is likely that most regulatory sequences lie within the IR. The IRs of several geminiviruses have been analyzed in the art and there has been searched for sequences involved in their replication and transcriptional regulation. By means of a phylogenetic-structural approach and based on available experimental evidence, it was postulated that iterative sequences found in this region could function as the determinants for replicative specificity of geminiviruses (Argüello-Astorga *et al*., 1994a; Argüello-Astorga *et al*., 1994b). Additionally, other sequences that could play a role in the expression of the viral ORFs were also found.

As mentioned above, geminiviruses cause important diseases in a number of crops. No effective control strategy has been developed to date. Due to economic importance of plant DNA viruses and, in particular, geminiviruses, there is a need for disease resistance strategy to be developed. Several genetic engineering strategies have been used based on coat protein expression, however these approaches are not effective against geminiviruses. Although conventional plant breeding has provided partial answers in a number of cases, the potential for an effective control strategy based on a transgenic approach remains very high. However there has been very little success in obtaining transgenic plants with an enhanced tolerance to geminiviral infection.
It is therefore an object of the current invention to propose a solution for this problem.
It appears, according to our invention, that the geminivirus AC2 gene product transactivates the expression of the coat and movement protein (CP and BV1) genes, and this effect seems to be mediated by specific although hitherto unknown *cis*-acting elements.
In accordance with the current invention it has been found that a truncated 115 nucleotide (nt) CP promoter sequence is responsive to a viral transactivator (SEQ.ID.NO.1). Transient gene expression and transgenic plant assays demonstrate that said truncated CP promoter sequence is unexpectedly still responsive to a viral transactivator. This promoter contains three elements similar to a sequence motif termed Conserved Late Element (CLE), according to the current invention called SEQ.ID.NO.2, SEQ.ID.NO.3 and SEQ.ID.NO.4 respectively, said motif is found in the regulatory regions of many geminiviruses being a potential functional target for AC2.

According to the current invention it is thus shown that some conserved elements (consensus : GTGGTCCC) present in the late gene promoters of several bipartite geminiviruses (thus termed Conserved Late Elements, CLEs), are functional target sites for the geminiviral transactivator. Thus herewith is disclosed that the participation of these elements in the AC2-mediated activation of gene expression is essential. An oligonucleotide containing two CLE motifs was synthesized (SEQ.ID.NO.5) and characterized in gain-of-function experiments. Transient expression assays showed that a 29 oligonucleotide sequence is still able to confer AC2- responsiveness to heterologous promoters. A smaller oligonucleotide (16 nt) containing a single CLE also conferred this activity (SEQ.ID.NO.6). In addition, when the CLE motifs were mutated in their original context (truncated 115 nt promoter), this modified promoter lost its ability to be transactivated by AC2.

In accordance with the invention an isolated DNA sequence is provided comprising the nucleotide sequence of SEQ. ID.NO.1 or functional fragments and/or any derivative thereof.
The isolated DNA sequence fragments are, for sake of clarity of the current description, the so-called CLE 1, CLE 2 or CLE 3 motifs as depicted in SEQ.ID.NO.2, SEQ.ID.NO.3 and SEQ.ID.NO.4 respectively.

Part of the invention is also an isolated DNA sequence comprising the nucleotide sequence of SEQ.ID.NO.5 or functional fragments and/or any derivative thereof, which sequence comprises two CLE motifs.

To the invention also belongs an isolated DNA sequence comprising the nucleotide sequence of SEQ.ID.NO.6 or functional fragments and/or any derivative thereof comprising only one CLE motif which forms the minimum requirement according to the invention still able to be transactivated upon geminiviral infection.

Furthermore a chimeric or recombinant DNA molecule comprising a geminivirus inducible promoter region having at least one of the DNA sequences above mentioned is another embodiment of the invention.

These chimeric or recombinant DNA molecules may further comprise a foreign DNA sequence, if expressed, results in plant cell death.

This so-called foreign DNA sequence may -for instance- be a suicide gene or any other means by which the plant defense mechanism is activated.

An important aspect of the current invention is a method for obtaining a plant with reduced susceptibility to a geminiviral infection and spread thereof comprising the steps of
a) transforming a recipient plant cell with a recombinant or chimeric DNA molecule according to the invention
b) generating a whole plant from a transformed cell and
c) identifying a transformed plant with said reduced susceptibility.

In addition a plant cell comprising said recombinant or chimeric DNA molecule and a plant obtainable by the method mentioned as well as plant material such as leaves, flowers, seeds, seedlings, roots, pollen or tubers obtainable from said plant belong to the scope of the instant invention.

Another aspect of the invention is a method for preventing geminivirus infection in a plant and subsequent outgrowth of said virus comprising planting a plant according to the invention in an area susceptible to geminiviral infection.

Another aspect of the current invention is the use of said promoter region comprising any of said DNA sequences to specifically arrest the spread or outgrowth of geminivirus in an infected plant as a result of specific killing the geminiviral infected cells. Alternatively the use of said promoter region comprising any of said DNA sequences also encompasses the feature that said promoter is only expressible when the cell is either infected with a geminivirus or part thereof of the same species or is infected with a geminivirus or part thereof of another species.

However it is nor necessary nor essential for the latter that the infecting virus contains a CLE region in order to act properly according to the invention.

Other applications for using said promoter region is the expression, under the control of said promoter, of specific proteins at the moment of infection. For instance the thus infected plant can produce specific compounds which can be used to determine the presence and/or activity of the infecting virus or alternatively the thus infected plant is able to produce a cytotoxic protein in order to obtain a hypersensitive response.

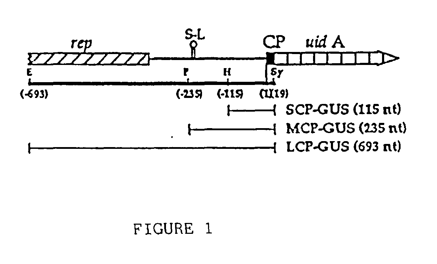

The CLE's are -as described- essential and sufficient to get transactivation by AC2. By the introduction of promoters (either the wild type CP promoter or a synthetic promoter containing one or more CLE's) into a plant (transgenic or transient experiments) detection of transactivation in these plants is feasible when AC2 is present. However it appears that in some tissues cellular (plant) proteins are present acting as AC2 and transactivate accordingly. This is apparently not good for the virus itself, so the virus has probably found an escape to this cellular activation by including a *silencer-like* element in its genome. With this silencer element the host proteins will not transactivate and the promoter will be turned on only when the AC2 protein is present. A "silencer" is understood as a promoter element that negatively regulate the transcription from the promoter. If the promoter is deleted the transcription is up-regulated inappropriately. Therefore the "silencer" is necessary for the correct activation of the promoter.
The above was observed with a construct that has been made containing a copy of the component A (2631 nt) as described in Figure 1A of Torres-Pacheco et al.,(1993) and the 115 nt CP promoter (SEQ.ID.NO.1). So a construct was obtained of 2746 bases (2631 and 115) wherein the 115 nt fragment was actually duplicated (present at both ends). Said *silencer-like* element is located around nt position number 2150 according to the sequence numbering in Figure 1A of Torres-Pacheco et al.,(1993).

In addition it is expected that a further refinement of the sequences according to the invention will lead to the identification of more specific sequences (so-called fragments) needed for the geminiviral-inducible promoter activity. In particular are interesting those sequences exhibiting structural features such as repeats and the like.

To the scope of the invention also belongs those variant promoter sequences obtained by modification including for instance exchange of a nucleotide for another nucleotide, inversions, deletions or insertions of a limited number of nucleotides remaining however the same specificity as the sequences described in SEQ ID NO's 1-6.

Promoters -optionally derivable from other viruses- with essentially similar sequence as the geminiviral-inducibie promoters according to the current invention, which have comparable or identical characteristics, can be isolated from other comparable species using the inventive promoter sequence(s) as, for instance, hybridization probe under conditions known to a skilled person. In another approach the nucleotide sequences or fragments thereof according to the invention can be used by skilled persons as so-called amplification primers in order to isolate promoter fragments with essentially similar sequences from other species by so-called amplification techniques like the PCR method.

In order to clarify what is meant in this description by some terms a further explanation is hereunder given.

The terms "polynucleotide", "DNA sequence", "nucleic acid sequence" or "nucleotide sequence" as used herein refers to a polymeric form of nucleotides of any length. This term refers only to the primary structure of the molecule. Thus, this term includes double- and single-stranded DNA. It also includes known types of modifications, for example, methylation, "caps" substitution of one or more of the naturally occuring nucleotides with analog.

"Transformation" as used herein, refers to the transfer of an exogenous polynucleotide into a host cell, irrespective of the method used for the transfer. The polynucleotide may be transiently or stably introduced into the host cell and may be maintained non-integrated , for example, as a plasmid, or alternatively, may be integrated into the host genome. Many types of vectors can be used to transform a plant cell and many methods to transform plants are available, Examples are direct gene transfer, pollen-mediated transformation, plant RNA virus-mediated transformation, *Agrobacterium*-mediated transformation, liposome mediated transformation, transformation using wounded or enzyme-degraded immature embryos, or wounded or enzyme-degraded embryogenic callus.

All these methods and several more are known to persons skilled in the art. The resulting transformed plant cell can then be used to regenerate a transformed plant in a manner known by a skilled person.

"Functional pad or fragment thereof" means that said part or fragment to which subject it relates has substantially the same activity as the subject itself, although the form, length or structure may vary.

With "recombinant DNA" or "chimeric DNA" is meant a hybrid DNA produced by joining pieces of DNA from different sources.

With "promoter" is meant a recognition site on a DNA strand to which RNA polymerase binds, thereby initiating transcription.

In the current invention is meant by "foreign DNA, foreign sequence or foreign gene" , a DNA sequence which is not in the same genomic environment (e.g. not operably linked to the same promoter and/or 3' end) in a plant cell, transformed with said DNA according to said invention, as is such DNA when it naturally occurs in a plant cell or the organism (bacterium, fungi, virus or the like) from which the DNA originates.

"Plant cell" comprises any cell derived from any plant and existing in culture as a single cell, a group of cells or a callus. A plant cell may also be any cell in a developing or mature plant in culture or growing in nature.

"Plants" comprises all plants, including monocotyledonous and dicotyledonous plants.

"Expression" means the production of a protein or nucleotide sequence in the cell itself or in a cell-free system. It includes transcription into an RNA product, post-transcriptional modification and/or translation to a protein product or polypeptide from a DNA encoding that product, as well as possible post-translational modifications.

In the description of the current invention reference is made to the following sequences of the Sequence Listing:
- SEQ.ID.NO. 1:: 1-115 nt fragment of the promoter sequence
- SEQ.ID.NO. 2:: so-called CLE-1 within SEQ.ID.NO.1
- SEQ.ID.NO. 3:: so-called CLE-2 within SEQ.ID.NO.1
- SEQ.ID.NO. 4:: so-called CLE-3 within SEQ.ID.NO.1
- SEQ.ID.NO. 5:: oligonucleotide sequence containing 2 x CLE-1 (of SEQ.ID.NO.2): 29 nt oligo
- SEQ.ID.NO.6:: oligonucleotide sequence containing 1 x CLE-1 (of SEQ.ID.NO.2): 16 nt oligo

A more detailed description of the invention, for the sake of clarity, is disclosed hereafter.

Transgenic tobacco plants harboring a construct with 115 nt of the CP promoter (SCP) fused to a GUS reporter gene according to the invention displayed blue spots consistently at the sites of impact when bombarded with PHV DNA (pepper huasteco virus). These plants normally do not express the transgene in most tissues. These results show that the *cis* acting elements involved in the transactivation process are located within the 115 bases upstream of the CP start codon, a region containing one CLE and two CLE-like motifs. To confirm that the CLEs are indeed responsible for the observed transactivation two other approaches were followed. First, the CLE motifs in the CP promoter were mutated. Although the modified promoter was still able to direct the expression of a reporter gene, no AC2-mediated transactivation was observed. Since one CLE and two inverted CLE-like sequences were all mutated, it is not possible to verify if only one or all of them are required for transactivation. In the second approach, a synthetic oligonucleotide that contains two perfect CLE motifs, conferred AC2-responsiveness to heterologous promoters. A smaller oligonucleotide (16 nt) that includes only a single CLE, was also able to mediate the transactivating effect of AC2 in the context of heterologous promoters. Since the CLE sequence is the only common motif shared between the 16 nt segment and the AC2-responsive truncated -115 CP promoter, therefore said element is the functional core-sequence involved in the transactivation process. A single CLE motif replacing an I-box element in a 52 nt region from the tobacco *rbcS* gene promoter, including the photoresponsive I-G unit (Argüello-Astorga and Herrera-Estrella, 1995), conferred AC2-responsiveness to a chimeric rbcS-35S promoter as determined by transient expression assays in pea leaf tissue.
Although the transactivation effect was observed with both AC2 sources (e.g., whole virus or an AC2-expressing cassette), a stronger expression is usually observed with the whole virus, even when the AC2 gene copy number delivered was similar. On one hand, it is possible that the virus delivered on the target cells can start replication, and thus, increase the AC2 gene copy number. However, other viral factors can also be directly or indirectly affecting CP expression. All CLE-less constructs show a lower expression than its equivalent CLE containing construct (e.g., 46GUS vs CLE46GUS). Nevertheless, the more profound difference in the expression levels obtained with the wt virus and the AC2-mutant agree with the fact that the AC2-mediated transactivation is the main mechanism for the increased expression of the reporter gene.

The CLE motif is not a sequence commonly found in plant gene promoters. A search among the upstream sequences of plant genes included in the GenBank database reported only a few matches. Interestingly, recent work with the rice PCNA gene promoter demonstrated the functionality of CLE-like sequences (Kosugi *et al*., 1995). In this work, it was reported that two elements highly homologous to CLEs (TGGTCCC and GTGGGCC), and a G-box were essential for the activity of the promoter in meristems. Thus, it is possible that CLE and CLE-like elements will reveal themselves as regulatory elements in plant promoters.

The 25 amino acid residues of the zinc finger-like domain is the most conserved protein segment of AC2 in subgroup III (SIII) geminiviruses, and it is also conserved in two subgroup II (SII) viruses, namely, the leafhopper-transmitted BCTV-CFH (but not the Logan, California and Worland isolates), and the treehopper-transmitted tomato pseudocurly top virus (TPCTV; Briddon *et al*., 1996). These are the only known SII geminiviruses that have CLE sequences. It has to be mentioned, however, that C2 in BCTV-Logan is not necessary for coat protein expression, suggesting a different role for this protein.

The present invention is further described by reference to the following non- limiting figures and examples.

Unless stated otherwise in the Examples, all recombinant DNA techniques are performed according to protocols as described in Sambrook et al. (1989), Molecular Cloning : A Laboratory Manual. Cold Spring Harbor Laboratory Press, NY or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, jointly published by BIOS Scientific Publications Ltd (UK) and Blackwell Scientific Publications (UK).

### Examples and description of materials and methods used.

### Sequence analysis

Sequences were assembled and analyzed using GeneWorks (Intelligenetics Inc.) and Lasergene (DNAStar) software packages. The following sequences were obtained from the EMBL and GenBank databases : African cassava mosaic virus Kenya (ACMV-K; accession num. X17095, X17096) and Malawi isolates (ACMV-M) (Hong *et al.,* 1993); Abutilon mosaic virus (AbMV; X15983, X15984); beet curly top virus (BCTV; X04144); bean golden mosaic virus, Puerto Rico (BGMV-PR; D00200, D00201), Brazil (BGMV-BZ; M88686, M88687), and Guatemala isolates (BGMV-GA; M91604, M91605); bean dwarf mosaic virus (BDMV; M88178, M88179); Indian cassava mosaic virus (ICMV; Z24758, Z24759); mung bean yellow mosaic virus (MYMV; D14703, D14704); pepper huasteco virus (PHV; X70418, X70419); Texas pepper virus (TPV; U57457); potato yellow mosaic virus (PYMV; D00940, D00941); squash leaf curl virus (SLCV; M38182, M38183); tomato golden mosaic virus (TGMV; K02029, M73794); tomato mottle virus (TMoV; L14460, L14461); tomato leaf curl virus, Indian (ToLCV-In; L12739) and Australian isolates (ToLCV-Au; Dry *et al.,* 1993); tomato yellow leaf cud virus, Israel (TYLCV-Is; X15656), Sardinia (TYLCV-Sr; X61153), and Thailand isolates (TYLCV-Th; M59838, M59839).

### Vector construction

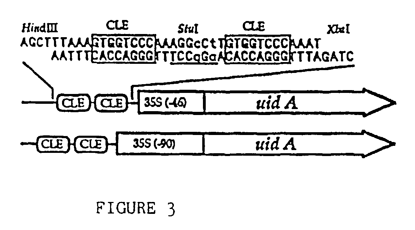

All DNA techniques were carried out according to standard procedures (Sambrook *et al.,* 1989) or as recommended by the suppliers. Full length clones of PHV (pepper huasteco virus) A component in pBluescript SK+ were obtained using either *Eco*RI (pIGV23) or *Hin*dIII (pIGV22) restriction enzymes (Torres-Pacheco *et al*., 1993). Translational fusions of the coat protein gene promoter with the *uid*A reporter gene (β-glucuronidase; GUS) were constructed by inserting the GUS-*nos* cassette from pBI101 into the *Sty*I-digested and blunt-ended pIGV23 (Bevan, 1984; Jefferson *et al.*, 1987). The *Sty*I digestion of pIGV23 eliminates most of the coat protein ORF leaving only the first 7 codons. Three versions (Large, Medium and Small) of the CP promoter were obtained by digesting with *Eco*RI, *Pm*/I or *Hin*dIII enzymes and fused to the GUS-*nos* cassette (see Figure 1).
The vectors used for AC2 transactivation assays were constructed as follows : Complementary oligonucleotides containing two CLE sequences (see Figure 3) were synthesized using an Applied Biosystems DNA Synthesizer. The oligonucleotides were designed in such a way that, when annealed, they generated *Hind*III (5') and *Xba*I (3') cohesive ends. The oligonucleotides (10 mM each) were annealed essentially as described by Sambrook *et al.* (1989). After annealing the oligonucleotides were inserted upstream of the CaMV 35S-GUS-*nos* fusions found in plasmids pBI46 and pBI90. pBI46 contains the minimal (nucleotides - 46/+8-) 35S promoter version, whereas pBI90 contains the truncated (-90/+1) version (Benfey *et al*, 1990). The newly generated plasmids were named pBI46CLE and pBI90CLE, respectively. These two plasmids were used to obtain transgenic tobacco plants. For transient assays, the CLE-35S (-46 or -90)-GUS-*nos* cassettes were subcloned into pBluescript SK+ to generate the plasmids CLE46GUS and CLE90GUS. To obtain a construct with a single CLE, CLE46GUS and CLE90GUS were digested with *Stu*I and *Hin*dIII (see Figure 3), filled-in and religated generating plasmid 1CLE46GUS and 1CLE90GUS. As bombardment controls, pBluescript SK+ harboring either the minimal or truncated promoters directing the expression of the GUS gene (46GUS and 90GUS, respectively) were used.

To mutate the CLE sequences in the CP promoter, the fragment *Hin*dIII-*Sty*I (SCP; -115 to +19) was synthetically constructed using two complementary oligonucleotides (89 and 87 bases) that overlapped 31 bases and included the mutated CLE sequences. The oligos were annealed, filled-in and ligated into pZero. The mutated *Hin*dIII-*Sty*I fragment was then excised and used to substitute the equivalent wild type fragment in the construct SCP-GUS. The GTGGTCC box found 18 nt upstream the ATG codon was changed to GATTACC. The CLE-like boxes GTGGTC and TTGGTCCC found in inverse orientation at 66 and 81 nt upstream the initiation codon were also changed to GTTTTC and TTTTACCC, respectively.

Two types of constructs were used as a source of AC2 product : plasmid pIGV22 (monomeric infectious clone of PHV A in pBluescript) and a construct where the expression of the AC2 ORF is directed by the 35S CaMV promoter (35S-AC2, also in pBluescript). To confirm AC2-specific effects, a nonsense mutation was introduced into the AC2 ORF of pIGV22 using a site directed mutagenesis kit, following the manufacturers' recommendations (Clontech; Palo Alto, CA). The oligonucleotide used: 5'-CGGCTGTTgAA**TTTAaA**TACATATTAACTG-3', introduced two changes (lower case) that produced two stop codons (underlined) and created a *Dra*I site (boldface). The mutations are located in the segment in which the AC2 ORF does not overlap with either AC3 nor AC1 ORFs.

### Transient assays

For transient expression experiments, a particle delivery system (model PDS1000, DuPont) was used to introduce the DNA of the constructs described above into pea or tobacco leaf tissue, following a procedure previously described (Klein *et al*, 1988). Before the bombardments leaf tissue was incubated for 4 hr at 25 °C on solid MS medium containing 0.4M mannitol and 0.4M sorbitol. The bombardments were carried out at a pressure of 800 lb. per square inch. Bombarded tissue was incubated at 25 °C for 18-24 h on solid MS medium without sucrose and then stained for glucuronidase activity (see below). The efficiency of bombardment was determined as the number of blue spots per bombardment per area.

### Histochemical staining

Histochemical staining for GUS activity either for transient expression assays or on transgenic tissue was done essentially as described by Jefferson *et al.,* 1987. Tissue was incubated at 37 °C for 4-8 h in a solution containing 0.5 mg/ml X-Gluc (Biosynth AG, Switzerland), 50 mM pH 7 phosphate buffer, 10 mM EDTA and 0.1% (V/V) Triton X-100. Stained tissue was cleared of pigments with several 30 min. washes using a 3:1 methanol-acetone mixture, until destaining was complete. The tissue and sections were visualized with a stereoscope (Nikon) or a bright field microscope (Zeiss).

### Plant inoculation

Tobacco plants were inoculated via particle bombardment as described before (Garzón-Tiznado *et al,* 1993). Plants were grown under greenhouse conditions at an average temperature of 28 °C.

### Plant transformation

Stable transformation of tobacco Xanthi nc plants was carried out by the leaf disc transformation method essentially as described by Horsch and Klee (1986).

### A 115 nt minimal PHV CP promoter is transactivated by AC2 protein

In order to delimit the sequences within the PHV CP promoter involved in the transactivation by AC2, the GUS reporter gene was fused to three different versions, in size, of the CP promoter, generating the fusions LCP-GUS (which includes 693 nt upstream from the CP start codon), MCP-GUS (235 nt) and SCP-GUS (115 nt) (Figure 1). Transient expression experiments assays showed that all versions of the CP promoter were able to be transactivated by a cloned PHV A component. Then, the shortest construct (SCP-GUS) was introduced into tobacco plants via *Agrobacterium tumefaciens* transformation. Histochemical analysis of primary transformants and T1 plants showed that the expression of the GUS reporter gene is restricted to vascular tissue of segments close to lateral buds and the apical portion of the stem. No GUS activity was detected in leaves or other tissues. These transgenic plants were inoculated via particle bombardment with PHV (Garzón-Tiznado *et al.*, 1993). Leaves and stems were then analyzed for GUS activity 2-3 weeks after inoculation, when symptoms were clearly visible. Inoculated leaves displayed strong glucuronidase activity mostly as discrete points (the sites of impact). In contrast, in mock-inoculated plants no such expression was detectable. These results confirmed that the sequences involved in transactivation by viral factors (most likely AC2) were present in the 115 nt fragment of the CP promoter (SCP).

### Conserved Late Element (CLE) sequences are involved in AC2 transactivation

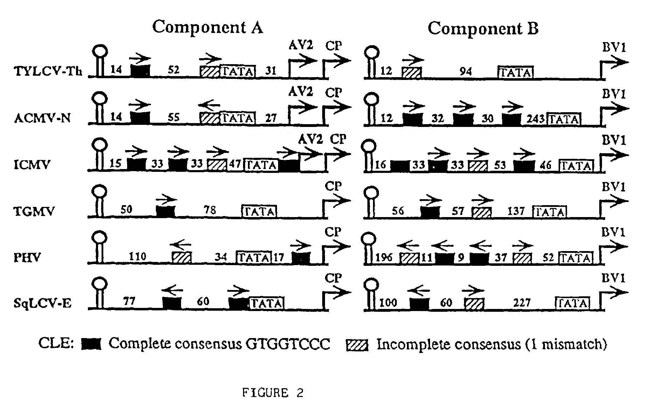

The 115 nt segment contains two elements highly similar to a conserved sequence motif (CLE) found in the promoters of late (i.e. CP and BV1) genes from all geminiviruses from the Old World (OW; Europe, Africa, and Asia) and some from the New World (NW; America), and postulated as a potential functional target for AC2 (Argüello-Astorga *et al*, 1994). Figure 2 shows the results of an analysis of the IR from selected OW and NW geminiviruses, in which the relative position and orientation of CLEs (one mismatch allowed) are indicated. In OW geminiviruses, a CLE is always present adjacent to the stem-loop element in both components, although component B generally contains more CLEs. The CLEs found in the NW viruses usually display more variation in their position within the IR.

To define if CLE sequences are indeed involved in the transactivation of PHV late genes, two approaches were followed. First, gain-of-function experiments with heterologous promoters were carried out. Initially, an oligonucleotide 29 bases in length that contains two CLE motifs was synthesized (Figure 3). A *Stu*I site was created between the CLE motifs to facilitate further assays with a promoter containing only one CLE. The synthetic oligonucleotide was cloned upstream of the minimal (nucleotides -46/+8) and truncated (nucleotides -90/+1) versions of the CaMV 35S promoter fused to the GUS reporter gene to generate the plasmids CLE46GUS and CLE90GUS, respectively (Figure 3).

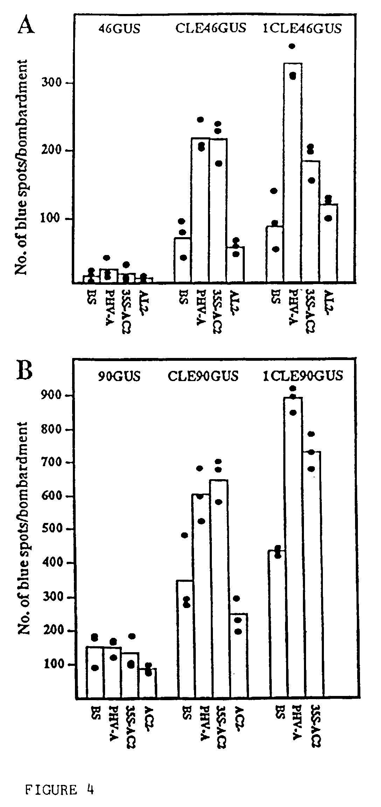

For the transient assays, leaf tissue from a PHV host (tobacco) and a non-host (pea) plant was bombarded with either 46GUS, 90GUS, CLE46GUS or CLE90GUS constructs in the absence or presence of a source of AC2 (either pIGV22 or p35S-AC2). The effect of AC2 was determined as the increase in the number of blue spots (GUS activity) found in the tissues co-bombarded with an AC2 source. As a negative control a PHV AC2 mutant (AC2-) was included in the experiments. Pea and tobacco leaf tissue bombarded with CLE46GUS and CLE90GUS in the absence or the presence of PHV A as a source of AC2 shows in both cases an increased intensity of the number of blue spots in the presence of PHV A DNA. Figures 4A and 4B show graphically the results of the bombardment experiments with tobacco leaves. No transactivation was observed in the experiments with the CLE-less 46GUS or 90GUS DNAs. The number of blue spots obtained was similar irrespective of the accompanying DNA (BS, PHV A, 35S-AC2 or AC2-). On the other hand, the co-bombardment of CLE46GUS (or CLE90GUS) with either PHV A or 35S-AC2 DNAs produced a higher expression of the reporter gene when compared with the controls that included DNA from pBluescript (BS) or the AC2-mutant.

Similar results were obtained with pea tissue, although in this case, a lower background and a higher transactivation level were usually observed. The results obtained clearly show that the 29 nt segment is able to confer AC2-responsiveness to heterologous promoters.

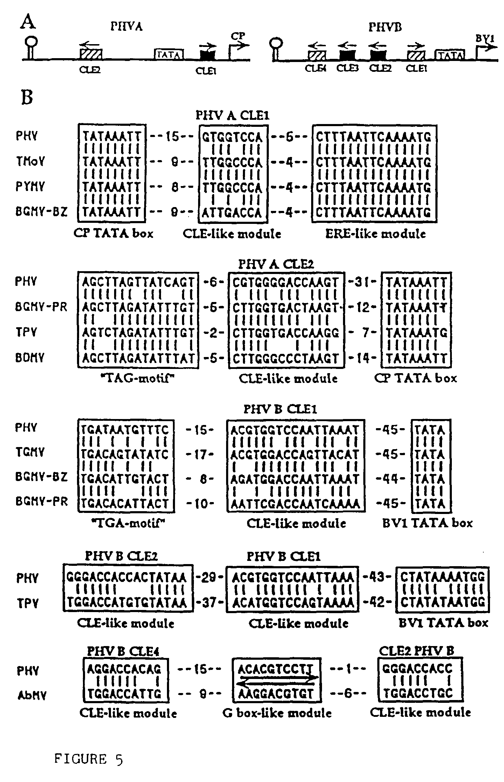

For the second approach, the CLE sequences found in the SCP-GUS construct were modified to disrupt the consensus. Transient experiments comparing the CLE-less SCP-GUS and the wt SCP-GUS showed a similar level of expression in the absence of a AC2 source. However, only the wt SCP-GUS was able to be transactivated when co-bombarded with a source of AC2 (Figure 5).

### A single CLE sequence is sufficient to transactivate heterologous promoters

To determine if a single CLE element was sufficient to confer AC2-responsiveness, the CLE46GUS and CLE90GUS constructs were digested with *Stu*I and *Hin*dIII to eliminate 13 nt of the original oligonucleotide including one CLE (see Figure 3). These new constructs, 1CLE46GUS and 1CLE90GUS, were also delivered onto tobacco leaves in the presence or absence of an AC2 source. The results obtained showed that a promoter with a single CLE was still able to be transactivated by AC2 (Figure 4). Again, a lower expression was observed when DNA from pBluescript or the AC2- PHV A mutant were co-bombarded. It was also noticed that the level of transactivation of these constructs with only 16 nt is similar to that obtained with the constructs harboring the complete 29 nt fragment (i.e. two CLEs).

### CLE sequence of PHV can also be induced by other wt geminiviruses

Tissues were bombarded with a construct containing the chimeric promoter including the CLE and the minimal 35S promoter (CLE46GUS).

This construct was co-bombarded with two versions of the component A of PHV (pIGV23 and pIGV22). Both transactivations were compared with a control wherein pBluescript DNA was used instead of viral DNA. In addition pTPVA, the component A of another geminivirus (Texas Pepper geminivirus, or recently renamed as Pepper golden mosaic virus) was used in the transactivation experiments and it appears that their activity is similar to the transactivation of PHV to the CLE of its own species. (see table 1).
Also the use of Taino tomato mottle virus results in a comparable transactivation.

**Table 1**

| Constructs | BsSK+ (pBluescript) | pIGV23 | pIGV22 | pTPVA |
|---|---|---|---|---|
| | not detectable | 140 | 200 | 150 |

The numbers indicate the quantity of blue spots observed on the leaves in the assay used as described above.

### References

Abouzid A.M., Frischmuth T, Jeske H (1988). A putative replicative form of the abutilon mosaic virus (gemini group) in a chromatin-like structure. Molecular and General Genetics, **212,**252-258.

Argüello-Astorga, G., Herrera-Estrella, L., and Rivera-Bustamante, R. (1994a).
Experimental and theoretical definition of geminivirus origin of replication. *Plant Molecular Biology* **26**, 553-556.

Argüello-Astorga, G. R., Guevara-González, R. G., Herrera-Estrella, L. R., and Rivera-Bustamante, R. F. (1994b). Geminivirus replication origins have a group-specific organization of iterative elements: A model for replication. *Virology* **203,** 90-100.

Argüello-Astorga, G., Herrera-Estrella, L. (1995). Theoretical and experimental definition of minimal photoresponsive elements in *cab* and *rbcS* genes. In: Terzi M, Cella R and Falavigna A (eds). Current issues in plant molecular and cellular biology. Kluwer Academic Publishers, Dordrecht, The Netherlands., pp.501-511.

Benfey, P. N., Ren, L., and Chua, N. H. (1990). Combinatorial and synergistic properties of CaMV 35S enhancer subdomains. *The EMBO Journal* **9**, 1685-1696.

Bevan, M. W. (1984). Binary *Agrobacterium* vectors for plant transformation. *Nucleic Acids Research* **12**, 8711-8720.

Briddon R.W, Bedford I.D, Tsai J.H, Markman P.G (1996). Analysis of the nucleotide sequence of the treehopper-transmitted geminivirus, tomato pseudo-curly top virus, suggests a recombinant origin. Virology,**219**: 387-394.

Davies, J. W., and Stanley, J. (1989). Geminivirus genes and vectors. *Trends in Genetics* **3**(3), 77-81.

Garzón-Tiznado, J. A., Torres-Pacheco, I., Ascencio-Ibañez, J. T., Herrera-Estrella, L., and Rivera-Bustamante, R. F. (1993). Inoculation of peppers with infectious clones of a new geminivirus by a biolistic procedure. *Phytopathology* **83**(5), 514-521.

Haley, A., Zhan, X., Richardson, K., Head, K., and Morris, B. (1992). Regulation of the activities of African cassava mosaic virus promoters by the AC1, AC2 and AC3 gene products. *Virology* **188**(2), 905-909.

Hong, Y. G., Robinson, D. J., and Harrison, B. D. (1993). Nucleotide sequence evidence for the occurrence of three distinct whitefly-transmitted geminiviruses in cassava, *Journal of General Virology* **74**(11), 2437-2443.

Horsch, R. B., and Klee, H. (1986). Rapid assay of foreign gene expression in leaf discs transformed by *Agrobacterium tumefaciens:* Role of T-DNA borders in the transfer process. *Proceedings of the National Academy of Sciences of the* USA **83**, 4428-4432.

Jefferson, R. A., Kavanaugh, T. A., and Bevan, M. W. (1987). GUS fusions: β-glucuronidase as a sensitive and versatile gene fusion marker in higher plants. *The EMBO Journal* **6**, 3901-3907.

Klein, T. M., Fromm, M. E., Weisinger, A., Tomes, A., Schaaf, D., Sletten, M., and Sanford, J. C. (1988). Transfer of foreign genes into intact maize cells using high-velocity microprojectiles. *Proceedings of the National Academy of Sciences of the USA* **85**, 4305-4309.

Kosugi, S., Suzuka, I., and Ohashi, Y. (1995). Two of three promoter elements identified in a rice gene for proliferating cell nuclear antigen are essential for meristematic tissue-specific expression. *The Plant Journal* **7**, 877-886.

Lazarowitz, S. G. (1992). Geminiviruses: Genome structure and gene function. *Critical Reviews in Plant Sciences* **11**(4), 327-349.

Martin, K. J., and Green, M. R (1992). Transcriptional activation by viral immediate early proteins: Variations on a common theme. In "Transcriptional Regulation" (S. L. McKnight, and K. R. Yamamoto, Eds.), pp. 695-725. Cold Spring Harbor Laboratory Press, Cold Spring Harbor, USA.

Nevins, J. R. (1991). Transcriptional activation by viral regulatory proteins. *Trends in Biochemical Sciences* **16**, 435-439.

Noris E, Jupin I, Accotto G.P, Gronenborn B. (1996). DNA binding activity of the C2 protein of tomato yellow leaf curl geminivirus. Virology,**217**,607-612.

Sambrook, J., Fritsch, E. F., and Maniatis, T. (1989). "Molecular Cloning. A laboratory manual." 2^{nd} Ed. 3 vols. Cold Spring Harbor Laboratory Press, Cold Spring Harbor, NY.

Sung Y.K, and Coutts R.H (1996). Potato yellow mosaic geminivirus AC2 protein is a sequence non-specific DNA binding protein. FEBS Letters,**383,** 51-54.

Sunter, G., and Bisaro, D. M. (1991). Transactivation of a geminivirus: AL2 gene product is needed for coat protein expression. *Virology* **180**, 416-419.

Sunter, G., and Bisaro, D. M. (1992). Transactivation in geminivirus AR1 and BR1 gene expression by the viral AL2 gene product occurs at the level of transcription. *Plant Cell* **4**(10), 1321-1331.

Sunter, G., and Bisaro, D.M. (1997). Regulation of a geminivirus coat protein by AL2 (TrAP): evidence for activation and derepression mechanisms. *Virology*, **232** (2), 269-280.

Sunter, G., Hartitz, M. D., and Bisaro, D. M. (1993). Tomato golden mosaic virus leftward gene expression: Autoregulation of geminivirus replication protein. *Virology* **195**(1), 275-280.

Sunter, G., Stenger, D. C., and Bisaro, D. M. (1994). Heterologous complementation by geminivirus AL2 and AL3 genes. *Virology* **203**(2), 203-210.

Timmermans, M. C. P., Prem Das, O., and Messing, J. (1994). Geminiviruses and their uses as extrachromosomal replicons. *Annual Review of Plant Physiology and Plant Molecular Biology* **45**, 79-112.

Torres-Pacheco, I., Garzón-Tiznado, J. A., Herrera-Estrella, L., and Rivera-Bustamante, R. F. (1993). Complete nucleotide sequence of pepper huasteco virus: Analysis and comparison with bipartite geminiviruses, *Journal of General Virology* **74**, 2225-2231.

### Legends to figures

**Figure 1**. Delimitation of the regions required for AC2-mediated transactivation. Schematic representation of the CP promoter versions used for stable plant transformation. Three versions of the PHV CP promoter were obtained by digesting with different enzymes. The size of each promoter is given as the distance between the 5' end and the CP start codon. (E = *Eco*RI, P = *Pm*/I, H = *Hin*dIII, St = *Sty*I, S-L = Stem-loop structure. The three versions were translationally fused to the *Escherichia coli* β-glucuronidase (*uid*A; GUS) coding sequence and the *nos* terminator.
**Figure 2**. Location and orientation of CLE sequences in selected geminiviruses.
   CLEs found in CP/AV2 and BV1 gene promoters from selected OW (TYLCV-Th, ACMV-N, and ICMV) and NW (PHV, SLCV-E and TGMV) geminiviruses. Filled-in boxes represent elements with the consensus sequence, whereas hatched boxes represent elements with one base mismatch. Relative orientation of the CLEs is indicated by the arrows.
**Figure 3**. CLE-containing heterologous promoters used in transient expression experiments.
   To determine the functionality of CLEs, oligonucleotides containing two CLEs were synthezised and cloned upstream of the minimal (-46/+8) and truncated (-90/+1) versions of the cauliflower mosaic virus (CaMV) 35S promoter to direct the expression of the reporter gene (GUS). The CLE sequences are boxed and the changes introduced to generate a *Stu*I site are shown in lower case.
**Figure 4**. Quantitative analysis of CLE-mediated transactivation in tobacco leaves.
   A) The constructs CLE46GUS, 1CLE46GUS, and 46GUS (control without CLE sequences) were co-bombarded into tobacco leaf tissue with two sources of AC2 (PHV A and 35S-AC2 DNAs). As controls, pBluescript DNA (BS), and DNA from a AC2 non-expressing PHV A mutant (AC2-) were also used. The number of blue spots in a given area were counted and plotted. Bars represent the average of the values obtained in three experiments; closed circles represent the values obtained in the individual experiments.
   B) A similar experiment was carried out with the constructs 90GUS (control), CLE90GUS, and 1 CLE90GUS.
**Figure 5**. A CLE-less PHV CP promoter is not transactivated.
   To verify the importance of the CLE motifs in the original context during the transactivation process, a CLE and two inverted CLE-like motifs were mutated. Wild type and CLE-less SCP-GUS constructs were co-bombarded into tobacco tissue in similar experiments as the one explained in Figure 4.

## Claims

1. An isolated DNA sequence comprising the nucleotide sequence of SEQ. ID.NO.1 or functional fragments and/or any derivative thereof.

2. Isolated DNA sequence fragments according to claim 1 wherein said fragments are CLE 1, CLE 2 or CLE 3 and are depicted in SEQ.ID.NO.2, SEQ.ID.NO.3 and SEQ.ID.NO.4 respectively.

3. An isolated DNA sequence comprising the nucleotide sequence of SEQ.ID.NO.5 or functional fragments and/or any derivative thereof which sequence comprises two CLE motifs.

4. An isolated DNA sequence comprising the nucleotide sequence of SEQ.ID.NO.6 or functional fragments and/or any derivative thereof comprising only one CLE motif.

5. Any of the constructs as outlined in Figure 1 or Figure 3.

6. A chimeric or recombinant DNA molecule comprising a geminivirus inducible promoter region having at least one of the DNA sequences of claims 1 to 4.

7. A chimeric or recombinant DNA molecule according to claim 6 further comprising foreign DNA, if expressed, results in plant cell death.

8. A chimeric or recombinant DNA molecule according to claim 7 wherein the foreign DNA is a suicide gene or any other means by which the plant defense mechanism is activated.

9. A method for obtaining a plant with reduced susceptibility to a geminiviral infection and spread thereof comprising the steps of
a) transforming a recipient plant cell with a recombinant or chimeric DNA molecule according to any of the claims 6-8
b) generating a whole plant from a transformed cell and
c) identifying a transformed plant with said reduced susceptibility.

10. A plant cell comprising a recombinant or chimeric DNA molecule according to any of the claims 6-8.

11. A plant obtainable by the method according to claim 9.

12. Plant material such as leaves, flowers, seed, seedlings, roots, pollen or tubers obtainable from the plant of claim 11.

13. A method for preventing geminivirus infection in a plant and subsequent outgrowth of said virus comprising planting a plant according to claim 11 in an area susceptible to geminiviral infection.

14. Use of a promoter region comprising the DNA sequence of any of the claims 1-4 to specifically arrest the spread or outgrowth of geminivirus in an infected plant or plant cell(s) as a consequence of specific killing the geminiviral infected cell(s).

15. Use of a promoter region comprising the DNA sequence of any of the claims 1-4 wherein said promoter is only expressible when the cell is either infected with a geminivirus or part thereof of the same species or is infected with a geminivirus or part thereof of another species.
